(19) Europäisches Patentamt
European Patent Office
Office européen des brevets

(11) **EP 1 559 775 A2**

(12) **EUROPÄISCHE PATENTANMELDUNG**

(43) Veröffentlichungstag:
**03.08.2005 Patentblatt 2005/31**

(51) Int Cl.[7]: **C12M 1/36**, C12G 1/02

(21) Anmeldenummer: **05001526.2**

(22) Anmeldetag: **26.01.2005**

(84) Benannte Vertragsstaaten:
**AT BE BG CH CY CZ DE DK EE ES FI FR GB GR HU IE IS IT LI LT LU MC NL PL PT RO SE SI SK TR**
Benannte Erstreckungsstaaten:
**AL BA HR LV MK YU**

(30) Priorität: **31.01.2004 DE 102004005495**

(71) Anmelder: **LiquosystemS GmbH**
**74366 Kirchheim (DE)**

(72) Erfinder: **Wieland, Stephan**
**74348 Lauffen (DE)**

(74) Vertreter: **Wörz, Volker**
**Dreiss, Fuhlendorf,**
**Steimle & Becker,**
**Postfach 10 37 62**
**70032 Stuttgart (DE)**

(54) **Verfahren zum kontrollierten Vergären einer Flüssigkeit**

(57) Die Erfindung betrifft ein Verfahren zum kontrollierten Vergären einer Flüssigkeit (3). Um bei dem Verfahren den Gärprozess besser beeinflussen und die Qualität der gegorenen Flüssigkeit (3) besser vorhersagen und reproduzieren zu können, wird vorgeschlagen, dass die Temperatur der Flüssigkeit (3) zumindest während eines Teils des Gärprozesses derart geregelt wird, dass ein vorgebbarer zeitlicher Dichteverlauf der Flüssigkeit (3) zumindest annähernd eingehalten wird. Insbesondere wird vorgeschlagen, dass der Gärprozess in drei Phasen unterteilt ist. Der Ablauf des gesamten Gärprozesses, insbesondere der Übergang zwischen den Phasen, erfolgt vollautomatisch.

*Fig. 1*

## Beschreibung

Die vorliegende Erfindung betrifft ein Verfahren zum kontrollierten Vergären einer Flüssigkeit.

Aus dem Stand der Technik sind Systeme zur Gärtemperaturregelung und zur Bestimmung der Dichte von Flüssigkeiten (sogenannten Dichtemesssonde) bekannt. Eine Dichtemesssonde ist beispielsweise aus der DE 199 63 935 A1 bekannt. Die Dichtemesssonde wird zum Messen der Konzentration einer Säure in Baumé-Graden oder das Mostgewicht eines Mostes oder Weines in Oechsle-Graden bestimmt. Das Mostgewicht ist weitgehend abhängig von dem Zuckergehalt des Mostes oder Weines, so dass das Mostgewicht zur überschlägigen Berechnung des Zuckergehaltes herangezogen werden kann. Das Mostgewicht in Oechsle kann nach folgender Gleichung bestimmt werden:

$$\text{Mostgewicht } [°\text{Oe}] = (\text{Dichte } 20/20 - 1) \cdot 1.000 \, [°\text{Oe}],$$

wobei für das Gewichtsverhältnis

$$\text{Dichte } 20/20 = \frac{\text{Dichte\_Most bei } 20°\text{C}}{\text{Dichte\_H2O bei } 20°\text{C}}$$

gilt.

Während des Gärvorgangs eines Mostes verringert sich dessen Dichte, da Zucker in Alkohol, Kohlendioxid, Säuren und zahlreiche andere Stoffe umgewandelt wird. Somit kann die Dichte einer gärenden Flüssigkeit (zum Beispiel Most oder Wein) und die zeitliche Änderung der Dichte einen zuverlässigen Hinweis auf den Zustand der Gärung geben.

Aus dem Stand der Technik ist es außerdem bekannt, durch Anheben oder Senken der Temperatur einer gärenden Flüssigkeit die Gäraktivität anzuregen bzw. zu hemmen. Dies wird bisher jedoch manuell gemacht, beziehungsweise erfolgt nicht mit dem Ziel, eine bestimmte Dichtezehrung (Abbau der Dichte der Flüssigkeit pro Zeiteinheit) zu erzielen. Außerdem beinhaltet die manuelle Beeinflussung der Gäraktivität - ohne automatische Überwachung derselben - das Risiko, dass die Gärung unter Umständen so weit verringert wird, dass sie zum erliegen kommt (Gärstokkung). Dies führt insbesondere bei der Weinvergärung zu einem sehr großen Qualitätsverlust.

Ausgehend von diesem Stand der Technik liegt der vorliegenden Erfindung die Aufgabe zu Grunde, ein Verfahren der eingangs genannten Art dahingehend auszugestalten und weiterzubilden, dass der Gärprozess besser beeinflusst und die Qualität der vergorenen bzw. gegorenen Flüssigkeit besser vorhergesagt und reproduziert werden kann.

Zur Lösung dieser Aufgabe wird ausgehend von dem Verfahren zum kontrollierten Vergären einer Flüssigkeit der eingangs genannten Art vorgeschlagen, dass die Temperatur der Flüssigkeit zumindest während eines Teils des Gärprozesses derart geregelt wird, dass ein vorgebbarer zeitlicher Verlauf mindestens einer den Gärzustand der Flüssigkeit charakterisierenden Größe zumindest annähernd eingehalten wird.

Als eine den Gärzustand der Flüssigkeit charakterisierende Größe wird vorzugsweise die Dichte der Flüssigkeit herangezogen. Es ist aber auch denkbar und Gegenstand der vorliegenden Erfindung, den Alkohol- und/oder Zuckergehalt und insbesondere alternativ oder zusätzlich den Kohlendioxid-Ausstoß aus der Flüssigkeit als eine den Gärzustand der Flüssigkeit charakterisierende Größe heranzuziehen. Anhand des Alkohol- oder Zuckergehalts und/ oder des Kohlendioxid-Ausstoßes aus der Flüssigkeit beziehungsweise anhand der zeitlichen Änderung dieser Größen kann über an sich bekannte Zusammenhänge mit guter Näherung auf die Dichte beziehungsweise Dichtezehrung der Flüssigkeit und damit auf den Zustand der Gärung geschlossen werden. Erfindungsgemäß wird also eine gezielte Beeinflussung der Gäraktivität der Flüssigkeit durch eine Temperaturregelung in Abhängigkeit mindestens einer beliebigen, den Gärzustand der Flüssigkeit charakterisierenden Größe vorgeschlagen.

Statt anhand des Kohlendioxid-Ausstoßes der Flüssigkeit auf die Dichte beziehungsweise auf die Dichtezehrung und daraus wiederum auf den Gärzustand der Flüssigkeit zu schließen, kann anhand des Kohlendioxid-Ausstoßes aus der Flüssigkeit auch direkt auf den Gärzustand der Flüssigkeit geschlossen werden.

Die Dichte der Flüssigkeit kann anhand einer Vielzahl von Größen hergeleitet werden. Entsprechende Größen sind beispielsweise der Zuckergehalt (beziehungsweise die Zuckerabnahme) der Alkohol-/Ethanolgehalt (beziehungsweise die Alkoholzunahme), das Glucose-/Fructose-Verhältnis, der Kohlendioxid-Ausstoß (beziehungsweise der Kohlendioxid-Strom), der Energieumsatz oder das Gewicht der Flüssigkeit.

Zur näherungsweisen Berechnung der Dichte der Flüssigkeit aus Zucker existieren unterschiedliche Gleichungen. Bei den nachfolgend beispielhaft angegebenen Gleichungen handelt sich größtenteils um lineare Umrechungen mit unterschiedlichen Korrekturwerten für den zuckerfreien Extrakt. Diese Gleichungen wurden zum Teil empirisch ermittelt.

Als Beispiel wird eine Näherungsformel angeführt, die lautet:

$$s = (a \cdot D) - 30$$

mit

s = Zucker in g/l,
a = Korrekturfaktor (liegt beispielsweise im Bereich von 2,61 oder 2,68),
D = Dichte in °Oe, und
30 = vermittelter Korrekturwert für den zuckerfreien Extrakt (liegt üblicherweise zwischen 20 und 30).

Für die Dichte D ergibt sich damit aus der obigen Gleichung:

$$D = \frac{s + 30}{a}.$$

Über Näherungsformeln lässt sich durch den Vergleich von Dichte- und Alkoholwerten und unter der Annahme eines Start-Alkoholgehalts von < 1 g/l zu Beginn der Gärung ein näherungsweiser Zusammenhang zwischen Dichtewerten und Alkoholgehalt während des Gärprozesses ableiten. Dabei kann beispielsweise die nachfolgende von der Anmelderin empirisch ermittelte Näherungsformel zur Ermittlung des Alkoholgehaltes eingesetzt werden:

$$A = F_1 \cdot D + F_2,$$

mit

A = Alkoholgehalt in g/l,
$F_1$ = Korrekturfaktor (nimmt üblicherweise Werte von ca. -1(g/l)/°Oe an),
$F_2$ = Korrekturwert abhängig von Ausgangsdichte zu Beginn der Gärung (üblicherweise 70 g/l < $F_2$ < 90 g/l), und
D = Dichte in °Oe,

wobei die Korrekturwerte $F_1$ und $F_2$ experimentell ermittelte Erfahrungswerte sind. Für die Dichte D ergibt sich aus obiger Gleichung somit:

$$D = \frac{a - F_2}{F_1}.$$

Mittels Näherungsformeln lässt sich über einen Vergleich von Dichtewerten mit dem Verhältnis von Glucose- zu Fructosewerten ein näherungsweise linearer Zusammenhang herleiten. Somit erlauben Fructose- und Glucosewerte Rückschlüsse auf den Dichteverlauf.

Die Menge des aus der Flüssigkeit entweichenden Kohlendioxids stellt ein Maß für die Gäraktivität der Flüssigkeit dar und lässt somit Rückschlüsse auf die Steigung der Zuckerkurve (und damit auf die Zuckerabnahme in der Flüssigkeit) zu. Bei einem bekannten Startzuckerwert zu Beginn des Gärprozesse kann näherungsweise der aktuelle Restzuckergehalt und daraus wiederum die aktuelle Ist-Dichte berechnet werden. Hierzu müssen folgende Startparameter bekannt sein:

- Dichte zu Beginn des Gärprozesses,
- Zuckergehalt zu Beginn des Gärprozesses, und
- Volumen der Flüssigkeit, deren Gäraktivität kontrolliert werden soll.

Die summarische Gärungsgleichung nach J. Gay-Lussac lautet:

$$1 \text{ Mol } C_6H_{12}O_6 \text{ (Glucose)} = 2 \text{ Mol } C_2H_5O_5 \text{ (Ethanol)} + 2 \text{ Mol } CO_2$$

und damit:

100 g Glucose = 51,5 g Alkohol + 48,5 g Kohlendioxid.

Somit entspricht die $CO_2$-Emission der Steigung der Zuckerabbaukurve. Nicht berücksichtigt sind hierbei allerdings der Zuckerverbrauch durch die Entwicklung von Biomasse (Hefen) sowie der Alkoholaustrag.

Bei einem bekannten Volumen der Flüssigkeit kann über das Gewicht der Flüssigkeit deren Dichte berechnet werden. Die Gleichung hierfür lautet:

$$D = m/V$$

mit

D = Dichte der Flüssigkeit in g/l,
m = Masse in g, und
V = Volumen der Flüssigkeit in 1.

Die verschiedenen Größen, anhand derer auf die Dichte der Flüssigkeit geschlossen werden kann, können auf beliebige Weise, entweder im Labor anhand einer Probe der Flüssigkeit oder anhand einer Online-Messung in der gärenden Flüssigkeit gemessen, ermittelt oder aus anderen Größen modelliert werden. Bei den nachfolgend beispielhaft aufgeführten Methoden zur Zuckerbestimmung handelt es sich um Labormethoden. Automatische, kontinuierliche Methoden zur Online-Messung des Zuckers in der gärenden Flüssigkeit sind derzeit zwar nicht bekannt, könnten jedoch während der Laufzeit des Patents noch entwickelt und dann im Rahmen des erfindungsgemäßen Verfahrens eingesetzt werden.

Zur Zuckerbestimmung kann eine der nachfolgenden an sich bekannten Methoden oder eine beliebig andere Methode eingesetzt werden: Verfahren nach Luff-Schoorl und Dr. Rebelein, enzymatische Zuckerbestimmung oder Infrarotspektroskopie (FTIR-Methode). Bei der FTIR-Methode handelt es sich um ein indirektes Messverfahren, bei welchem ein Wellenmuster (Interferogramm), das die gesamte Information des Infrarotspektrums enthält, mit vorher kalibrierten Referenzwerten aus repräsentativen Mustern verglichen wird. Dieses Verfahren beinhaltet also eine rechnerische Bearbeitung spezifischer Molekülschwingungsspektren.

Bei den nachfolgend beispielhaft aufgeführten Methoden zur Alkoholbestimmung handelt es sich ebenfalls um Labormethoden. Automatische, kontinuierliche Verfahren zur Online-Messung des Alkoholgehalts in gärenden Flüssigkeiten sind derzeit noch nicht bekannt. Solche Verfahren zur Online-Messung könnten jedoch durchaus während der Laufzeit des Patents entwickelt und im Rahmen des erfindungsgemäßen Verfahrens eingesetzt werden.

Zur Alkoholbestimmung kann eine der nachfolgenden an sich bekannten Methoden oder eine beliebig andere Methode eingesetzt werden: Destillationsmethode, Verfahren nach Dr. Rebelein, enzymatische Alkoholbestimmung, Gaschromatographie oder Infrarotspektroskopie (FTIR-Methode). Eine Alkoholbestimmung mit Biosensoren ist ebenfalls denkbar. Biosensoren sind derzeit jedoch noch relativ wartungsintensiv und besitzen eine geringe Robustheit, die den Einsatz dieser Technik im Weinbereich noch unwirtschaftlich machen. Bereits in wenigen Jahren könnten jedoch die Alkoholbestimmung mit Biosensoren so weit fortentwickelt sein, dass sie im Rahmen des erfindungsgemäßen Verfahrens eingesetzt werden kann.

Zur Bestimmung des Kohlendioxid-Ausstoßes aus der Flüssigkeit kann eine der nachfolgenden an sich bekannten Methoden zur Massen- oder Volumenstrommessung oder eine beliebig andere Methode eingesetzt werden: $CO_2$-Messung nach Saller, Wärmeleitfähigkeitsmessung, mikrothermische Durchflussmessung, Gas-Mengenmessung mittels eines mechanischen Gerätes, beispielsweise mittels eines Wirbelstrommessgerätes, Geschwindigkeitsmessung der vorbeiströmenden Gasmengen mittels eines Flügelradsensors (sogenanntes Anemometer), Staurohrmessung oder optische Messung.

Über die bei der Gärung freigesetzte Wärmeleistung wird auf die Gäraktivität und somit auf den Zuckerabbau geschlossen. Bei einem bekannten Zuckergehalt zu Beginn des Gärprozesses kann somit der aktuelle Zuckergehalt und daraus die Dichte der Flüssigkeit ermittelt werden. Bei der Messung der Wärmefreisatzes beziehungsweise des Energieumsatzes wird die Tanktemperatur und die Einlauf- und Auslauftemperatur des Kühlmittels unter Berücksichtigung der Menge, des Durchsatzes und der Art des Kühlmittels, und des Wärmeverlustes durch Abstrahlung an den Raum und des Verlustes durch $CO_2$-Ausgasung erfasst.

Zum Messen des Gewichts der Flüssigkeit kann entweder eine Probe mit einem definierten Volumen oder der komplette Tank oder Behälter, der die zu gärende Flüssigkeit enthält, abgewogen werden. Beim Abwiegen einer Probe wird durch eine Entnahmevorrichtung dem Tank ein definiertes Flüssigkeitsvolumen entnommen. Die Gewichtsbestimmung kann durch eine gängige Waage mit hoher Auflösung erfolgen. Beim Wiegen des gesamten Tanks oder Behälters ist der komplette Flüssigkeitsbehälter auf einer oder mehreren Waagen platziert, die kontinuierlich das Gesamtgewicht des Behälters ermitteln. Zusätzlich zum Flüssigkeitsgewicht muss das Volumen der Flüssigkeit messtechnisch erfasst werden. Dies erfolgt in der Regel durch eine Füllstandsmessung (zum Beispiel mit Radarsensoren) bei bekannter Tankgeometrie. Bei bekanntem Leergewicht des Behälters und bekanntem Flüssigkeitsvolumen kann die Dichte aus dem Gewicht der Flüssigkeit hergeleitet werden.

Erfindungsgemäß wird insbesondere eine dichte- oder kohlendioxidabhängige Temperaturregelung und damit eine gezielte Beeinflussung der Hefeaktivität in der gärenden Flüssigkeit vorgeschlagen. Der vorgebbare zeitliche Dichteverlauf beziehungsweise Kohlendioxid-Ausstoß kann entweder anhand von Berechnungen oder empirisch ermittelt werden. Der Dichteverlauf beziehungsweise Kohlendioxid-Ausstoß kann in Form einer Kennlinie oder eines Kennfeldes oder in Form einer mathematischen Funktion vorliegen. Der Ablauf des Gärprozesses kann bedienerfreundlich mittels eines geeigneten Computerprogramms mit einer grafischen Benutzerschnittstelle (GUI) voreingestellt, beobachtet und beeinflusst werden.

Durch das erfindungsgemäße Verfahren kann während des Gärprozesses ein definierter Abbau der Dichte pro Zeiteinheit (sogenannte Dichtezehrung) beziehungsweise Kohlendioxid-Strom pro Zeiteinheit erzielt werden. Dadurch kann die Gesamtgärdauer vorhergesagt und ein vorgegebener Gärverlauf eingehalten werden. Mit dem erfindungsgemäßen Verfahren können reintönigere Weine mit einem verbesserten Primäraroma erzeugt werden. Außerdem kann ein definierter Endvergärungsgrad oder - falls gewünscht - eine vollständige Durchgärung der Flüssigkeit erzielt werden. Schließlich kann auch eine Zeitersparnis bei der Vergärung erzielt werden.

Durch den vollautomatischen Ablauf des erfindungsgemäßen Verfahrens wird die Fehleranfälligkeit reduziert und Arbeitskapazität eingespart. Außerdem kann durch eine gezielte Beeinflussung der Dichtezehrung beziehungsweise des Kohlendioxid-Stroms die Qualität (Zusammensetzung, Aussehen, Geschmack, etc.) der gärenden Flüssigkeit beeinflusst werden. Dadurch kann eine Prozessoptimierung des Gärprozesses, eine Erhöhung der Prozesssicherheit und eine Qualitätssteigerung der gegorenen bzw. vergorenen Flüssigkeit erzielt werden.

Gemäß einer vorteilhaften Weiterbildung der Erfindung wird vorgeschlagen, dass die Temperatur der Flüssigkeit innerhalb eines vorgebbaren Temperaturbereichs zwischen einer minimalen Temperatur und einer maximalen Temperatur geregelt wird. Die Temperatur als Stellgröße darf sich nur innerhalb des vorgegebenen Temperaturbereichs bewegen. Die minimale und maximale Temperatur muss kein über die Dauer des gesamten Gärprozesses konstanter Wert sein, sondern kann sich beispielsweise zeitabhängig oder beim Vorliegen bestimmter Bedingungen oder Ereignisse verändern. Die Werte für die minimale und maximale Temperatur ergeben sich beispielsweise aus den Randbedingungen für die Aufrechterhaltung des Gärprozesses oder für die Erzielung einer bestimmten Qualität der gegorenen Flüssigkeit. Auf diese Weise kann ein ungewollter Abbruch des Gärvorgangs (sogenannte Gärstockung) oder eine Beeinträchtigung der Qualität der gegorenen bzw. vergorenen Flüssigkeit sicher und zuverlässig vermieden werden.

Der Gärprozess ist vorzugsweise in verschiedene Phasen unterteilt. Denkbar ist beispielsweise eine Unterteilung in eine Angärphase, eine Hauptgärphase und eine Endgärphase. Sobald bestimmte Randbedingungen erfüllt sind, wird automatisch von der einen Phase in die nächste Phase gewechselt. Gemäß einer bevorzugten Ausführungsform der Erfindung wird vorgeschlagen, dass während der Angärphase zu Beginn des Gärprozesses die Temperatur der Flüssigkeit auf einen vorgebbaren konstanten Wert geregelt wird, bis sich in der Flüssigkeit ein vorgebbarer Dichteabbau beziehungsweise Kohlendioxid-Strom pro Zeiteinheit einstellt, bis ein vorgebbarer absoluter Dichteabbau beziehungsweise Kohlendioxid-Strom stattgefunden hat oder bis eine vorgebbare Zeitdauer überschritten ist. In der Angärphase greift das System, ausgehend von einer vorgebbaren, vorzugsweise konstanten Gärstart-Temperatur zu einem bestimmten Zeitpunkt, bei dem vorzugsweise eine vorgebbare optimale Dichtezehrung (Gärintensität) beziehungsweise ein optimaler Kohlendioxid-Strom für die Einleitung der Hauptphase vorliegt, in den Gärprozess ein. Während der Angärphase wird also der Dichtabbau beziehungsweise der Kohlendioxid-Strom überwacht und der optimale Zeitpunkt für die Einleitung der Hauptgärphase automatisch erkannt. Wird die optimale Dichtezehrung beziehungsweise der optimale Kohlendioxid-Strom innerhalb einer vorgebbaren Zeitdauer oder innerhalb des vorgebbaren absoluten Dichteabbauwertes beziehungsweise Kohlendioxid-Stromwertes nicht erreicht, wird nach Erreichen eines der vorgebbaren Grenzwerte (Ablauf der Zeitdauer bzw. Erreichen des Dichteabbauwertes beziehungsweise Kohlendioxid-Stromwertes) trotzdem in die Hauptgärphase umgeschaltet.

Vorteilhafterweise wird nach der Angärphase automatisch in eine Hauptgärphase übergegangen, während der die Temperatur der Flüssigkeit derart geregelt wird, dass sich in der Flüssigkeit ein vorgebbarer Dichteabbau beziehungsweise Kohlendioxid-Strom pro Zeiteinheit einstellt. Während der Hauptgärphase ermöglichen also automatische Temperaturänderungen der Flüssigkeit eine schonende, vorgegebene Dichtezehrung beziehungsweise einen schonenden, vorgegebenen Kohlendioxid-Strom. Neben der optimalen Streckung oder Raffung des Gärverlaufs zur Erzeugung bestimmter Weinstile ergibt sich eine planbare Gärdauer.

Vorzugsweise wird aus der Hauptgärphase automatisch in eine Endgärphase übergegangen, sobald ein vorgebbarer Schwellwert für die Dichte beziehungsweise für die gesamte aus der Flüssigkeit abgegebene Kohlendioxid-Menge oder eine vorgebbare Zeitdauer überschritten ist. Dies sind die Umschaltbedingungen für den Übergang aus der Hauptgärphase in die Endgärphase. Die Vorgabe verschiedener Schwellwerte für die Dichte beziehungsweise Kohlendioxid-Menge der Flüssigkeit kann die Qualität eines Weines deutlich beeinflussen.

Es wird vorgeschlagen, dass während der Endgärphase gegen Ende des Gärprozesses die Temperatur der Flüssigkeit derart geregelt wird, dass sich in der Flüssigkeit ein Dichteabbau beziehungsweise ein Kohlendioxid-Strom pro Zeiteinheit von etwa Null einstellt und ein vorgebbarer Endwert für die Dichte beziehungsweise für die gesamte abgegebene Kohlendioxid-Menge der Flüssigkeit erreicht wird. In der Endgärphase kann also ein automatisches Abstoppen des Gärprozesses auf einen absoluten, vorgebbaren Dichte- beziehungsweise Kohlendioxid-Endwert, beispielsweise zur Herstellung fructosebetonter Weine, durch geeignete Temperaturregelung erzielt werden. Die Temperaturregelung erfolgt dabei innerhalb eines Temperaturkorridors, der von dem zulässigen Temperaturbereich während der Angärphase und der Hauptgärphase abweichen kann. Dabei wird die Regelung nach Dichtezehrung beziehungsweise Kohlendioxid-Strom beendet.

Vorteilhafterweise wird die Temperatur der Flüssigkeit während dieser Ausführungsform der Endgärphase innerhalb eines vorgebbaren End-Temperaturbereichs zwischen einer minimalen End-Temperatur und einer maximalen End-Temperatur geregelt. Vorzugsweise liegt dabei die minimale End-Temperatur niedriger als die minimale Temperatur und liegt die maximale End-Temperatur niedriger als die maximale Temperatur.

Alternativ wird vorgeschlagen, dass während der Endgärphase gegen Ende des Gärprozesses die Temperatur der Flüssigkeit derart geregelt wird, dass sich eine vollständige Durchgärung der Flüssigkeit einstellt. Vorzugsweise wird dabei mit der Dichtezehrung beziehungsweise Kohlendioxid-Strom aus der Hauptgärphase weitergearbeitet, das heißt die Regelung nach Dichtezehrung wird fortgesetzt. Selbstverständlich muss dabei nicht auf die gleichen Parameterwerte zugriffen werden wie während der Hauptgärphase. Es ist durchaus denkbar, dass für die Regelung nach Dichtezehrung beziehungsweise Kohlendioxid-Strom während der Endgärphase neue, gegenüber den Parameterwerten während der Hauptgärphase veränderte Parameterwerte vorgegeben werden.

Vorteilhafterweise wird die Temperatur der Flüssigkeit während der alternativen Ausführungsform der Endgärphase innerhalb eines vorgebbaren End-Temperaturbereichs zwischen einer minimalen End-Temperatur und einer maximalen End-Temperatur geregelt. Vorzugsweise liegt die minimale End-Temperatur dabei höher als die minimale Temperatur und liegt die maximale End-Temperatur niedriger als die maximale Temperatur.

Zur Erhöhung der Prozesssicherheit wird ein Benachrichtigungsoder Alarmierungssystem eingesetzt, das bei Erreichen von vorgebbaren Schwellwerten oder beim Übergang von einer Gärphase in eine andere den Anwender über den aktuellen Zustand des Gärprozesses informiert. Die Schwellwerte betreffen beispielsweise eine minimal und maximal zulässige Dichtezehrung, einen minimal und maximal zulässigen Kohlendioxid-Strom, eine minimal und maximal zulässige Temperatur der Flüssigkeit und/oder eine minimal und maximal zulässige Endgärdichte. Die Information des Anwenders erfolgt beispielsweise über die Ausgabe entsprechender Meldungen oder Signalverläufe auf einem Bildschirm eines Steuergeräts oder ähnlichem. Es ist auch an eine Information des Anwenders über den Prozesszustand anhand einfacher Leuchtsignale (Warn- oder Zustandsleuchten) oder Akustiksignale gedacht.

Von besonderer Bedeutung ist die Realisierung des erfindungsgemäßen Verfahrens in der Form eines Computerprogramms. Dabei ist das Computerprogramm auf einem Rechengerät, insbesondere auf einem Mikroprozessor, ablauffähig und zur Ausführung des erfindungsgemäßen Verfahrens programmiert. In diesem Fall wird also die Erfindung durch das Computerprogramm realisiert, so dass dieses Computerprogramm in gleicher Weise die Erfindung darstellt wie das Verfahren, zu dessen Ausführung das Computerprogramm programmiert ist. Das Computerprogramm ist vorzugsweise auf einem Speicherelement abgespeichert. Als Speicherelement kann insbesondere ein elektrisches, optisches oder magnetisches Speichermedium zur Anwendung kommen, beispielsweise ein Random-Access-Speicher, ein Read-Only-Speicher, ein Flash-Speicher, eine Diskette, eine CD-ROM oder eine DVD.

Außerdem wird erfindungsgemäß ein Steuergerät für eine Temperaturregelung einer Flüssigkeit vorgeschlagen, das ein Rechengerät, insbesondere einen Mikroprozessor, und ein Speicherelement umfasst. Erfindungsgemäß wird vorgeschlagen, dass auf dem Speicherelement ein Computerprogramm abgespeichert ist, das zur Ausführung des erfindungsgemäßen Verfahrens programmiert ist und das zur Ausführung des Verfahrens auf dem Rechengerät abläuft. Das Steuergerät kann Teil einer speicherprogrammierbaren Steuerung (SPS) oder ein herkömmlicher Computer (PC) sein.

Weitere Merkmale, Anwendungsmöglichkeiten und Vorteile der Erfindung ergeben sich aus der nachfolgenden Beschreibung von Ausführungsbeispielen der Erfindung, die in der Zeichnung dargestellt sind. Dabei bilden alle beschriebenen oder dargestellten Merkmale für sich oder in beliebiger Kombination den Gegenstand der Erfindung, unabhängig von ihrer Zusammenfassung in den Patentansprüchen oder deren Rückbeziehung sowie unabhängig von ihrer Formulierung bzw. Darstellung in der Beschreibung bzw. in der Zeichnung. Es zeigen:

Figur 1    eine Vorrichtung zur Ausführung eines erfindungsgemäßen Verfahrens gemäß einer bevorzugten Ausführungsform;

Figur 2    einen Signalverlauf des gesamten erfindungsgemäßen Verfahrens gemäß einer bevorzugten Ausführungsform;

Figur 3    eine graphische Benutzerschnittstelle zur Parametrierung des erfindungsgemäßen Verfahrens vor dessen Ausführung;

Figur 4    einen Signalverlauf des erfindungsgemäßen Verfahrens aus Figur 2 während einer Angärphase;

Figur 5    einen Signalverlauf des erfindungsgemäßen Verfahrens aus Figur 2 während einer Hauptgärphase;

Figur 6    einen Signalverlauf des erfindungsgemäßen Verfahrens aus Figur 2 während einer Endgärphase gemäß einer ersten Ausführungsform; und

Figur 7    einen Signalverlauf des erfindungsgemäßen Verfahrens aus Figur 2 während einer Endgärphase gemäß einer zweiten Ausführungsform.

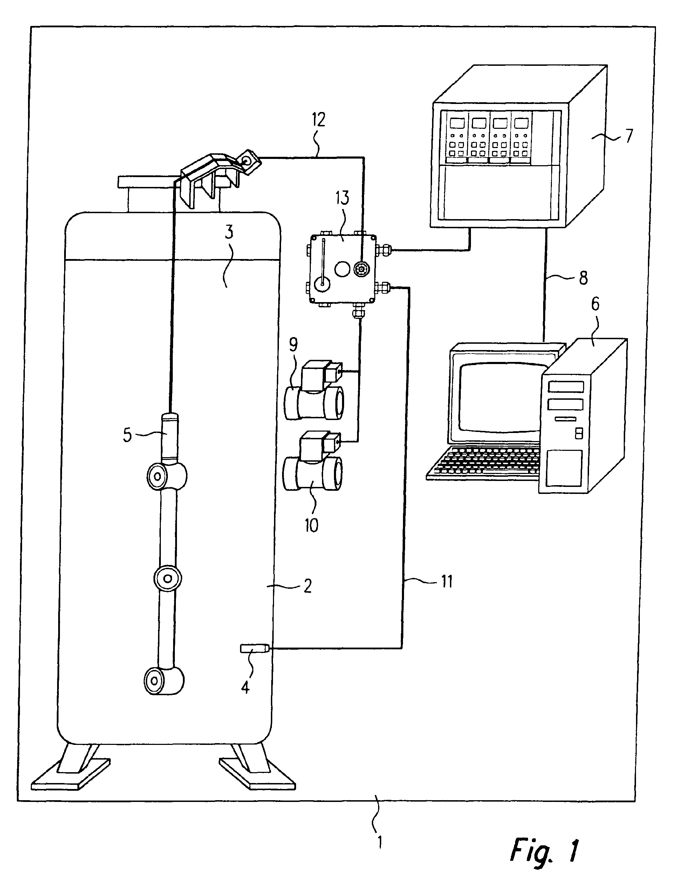

In Figur 1 ist eine Vorrichtung zur Ausführung eines erfindungsgemäßen Verfahrens gemäß einer bevorzugten Ausführungsform in seiner Gesamtheit mit dem Bezugszeichen 1 bezeichnet. Die Vorrichtung 1 umfasst einen Flüssigkeitsbehälter 2, der zum Teil mit der zu gärenden Flüssigkeit 3 gefüllt ist. Die Flüssigkeit 3 ist beispielsweise Traubensaft bzw. Wein. In die Flüssigkeit 3 ist in einem seitlichen Bereich des Behälters 2 ein Temperaturfühler 4 zum Messen der Temperatur der Flüssigkeit 3 getaucht. Außerdem ist von oben eine Dichtemesssonde 5 zum Erfassen der Dichte der Flüssigkeit 3 in diese getaucht. Nähere Angaben über den Aufbau und die Funktionsweise der Dichtemesssonde 5 können der DE 199 63 935 A1 entnommen werden. Die Dichtemesssonde 5 wird in dem dargestellten Ausführungsbeispiel zum Messen des Mostgewichts der Flüssigkeit 3 in Oechsle-Graden eingesetzt.

Selbstverständlich kann die Dichte der Flüssigkeit 3 auch auf andere Weise als mit einer Dichtesonde bestimmt werden. Vorzugsweise wird die Dichte anhand anderer Größen der Flüssigkeit 3, ganz besonders bevorzugt anhand des Gärzustandes der Flüssigkeit 3 charakterisierenden Größen, ermittelt oder modelliert. Solche Größen sind beispielsweise der Alkohol- oder Zuckergehalt der Flüssigkeit 3 oder der Kohlendioxid-Ausstoß aus der Flüssigkeit 3. Diese Größen können auf beliebige, an sich bekannte Art und Weise ermittelt, gemessen oder modelliert werden. Aus diesen Größen kann anhand beliebiger, an sich bekannter Verfahren die Dichte der Flüssigkeit 3 hergeleitet und der aktuelle Zustand der Gärung ermittelt werden.

Während des Gärvorgangs des Weines verringert sich dessen Dichte, da Zucker in Alkohol, Kohlendioxid, Säuren und andere Stoffe umgewandelt wird. Somit kann die Dichte des Weines und die Änderungsgeschwindigkeit der Dichte einen zuverlässigen Hinweis auf den Zustand der Gärung geben.

Die Vorrichtung 1 umfasst außerdem ein Steuergerät, das in dem vorliegenden Ausführungsbeispiel einen Personal Computer (PC) 6 und eine speicherprogrammierbare Steuerung (SPS) 7 umfasst. Der PC 6 umfasst Benutzerschnittstellen in Form eines Bildschirms und einer Tastatur. Außerdem umfasst der PC 6 ein Rechengerät, das insbesondere als ein Mikroprozessor (nicht dargestellt) ausgebildet ist. Schließlich umfasst der PC 6 auch ein elektrisches Speichermedium (nicht dargestellt), das beispielsweise als ein Random-Access-Speicher, als ein Read-Only-Speicher oder als ein Flash-Speicher ausgebildet ist. Auf dem Speichermedium ist ein Computerprogramm zur Ausführung des erfindungsgemäßen Verfahrens abgespeichert. Zur Abarbeitung des Computerprogramms wird es entweder abschnittsweise oder als ganzes an den Mikroprozessor übertragen und dort abgearbeitet. Der PC 6 und die Mikroprozessorsteuerung bzw. die SPS 7 stehen über eine Datenverbindung 8 miteinander in Verbindung.

Selbstverständlich kann statt der separaten SPS 7, deren Funktion auch in das Computerprogramm integriert sein, so dass als Steuergerät ausschließlich der PC 6 eingesetzt wird. Die Funktion der SPS 7 wird dann von dem PC 6 übernommen. Das in diesem Fall dann auf dem PC 6 ablaufende Computerprogramm müsste auch die in dem in Figur 1 dargestellten Ausführungsbeispiel durch die SPS 7 realisierten Funktionen erfüllen können. Alternativ könnte die Funktion der SPS 7 auch in einem mikrocontrollergesteuerten Regler enthalten sein.

Bei dem in Figur 1 dargestellten Ausführungsbeispiel umfasst das auf dem PC 6 ablauffähige Computerprogramm die Funktionen Parametrierung des Temperaturreglers, graphische Datenaufzeichnung und Ausgabe von Meldungen, Signalverläufen, etc. Das Computerprogramm wird entweder als solches über das Internet oder gespeichert auf einem Datenträger vertrieben.

Bei dem Ausführungsbeispiel ist in der SPS 7 eine Ablaufsteuerung, eine Temperaturregelung, eine Messdatenerfassung und eine Prozessüberwachung integriert. Zur Temperaturregelung ist in die SPS 7 ein Temperaturregler integriert, durch den die Temperatur der Flüssigkeit 3 geregelt werden kann. Dazu sind Heizmittel und Kühlmittel (beide nicht dargestellt) vorgesehen, die temperaturerhöhend bzw. temperaturverringernd auf die Flüssigkeit 3 einwirken. Die Heiz- und Kühlmittel arbeiten elektrisch oder hydraulisch über eine Heiz- bzw. Kühlflüssigkeit. In dem dargestellten Ausführungsbeispiel umfassen die Kühlmittel einen Heizkreislauf und einen Kühlkreislauf, die unabhängig voneinander über geeignete elektromagnetisch betätigbare Ventile 9, 10 aktiviert und deaktiviert werden können. Die SPS 7 kann durch Ansteuern der Ventile 9, 10 die Heizmittel bzw. die Kühlmittel aktivieren und auf diese Weise die Temperatur der Flüssigkeit 3 auf einen vorgebbaren Wert einstellen. Durch Anheben oder Senken der Temperatur der gärenden Flüssigkeit 3 kann die Gäraktivität angeregt bzw. gehemmt werden.

Über eine erste Messdatenleitung 11 steht der Temperaturfühler 4 mit der SPS 7 in Verbindung. Die gemessene Temperatur der Flüssigkeit 3 wird an die SPS 7 geführt und für die Temperaturregelung herangezogen. Über eine zweite Messdatenleitung 12 steht die Dichtemesssonde 5 mit der SPS 7 in Verbindung. Die gemessene Dichte und damit eine den Gärvorgang charakterisierende Größe wird über die Messdatenleitung 12 an die SPS 7 geführt und ebenfalls für die Temperaturregelung herangezogen. Die beiden Messdatenleitungen 11, 12 sind über eine Verklemmdose 13 an die SPS 7 angeschlossen. Selbstverständlich können sie auch direkt an die SPS 7 angeschlossen sein.

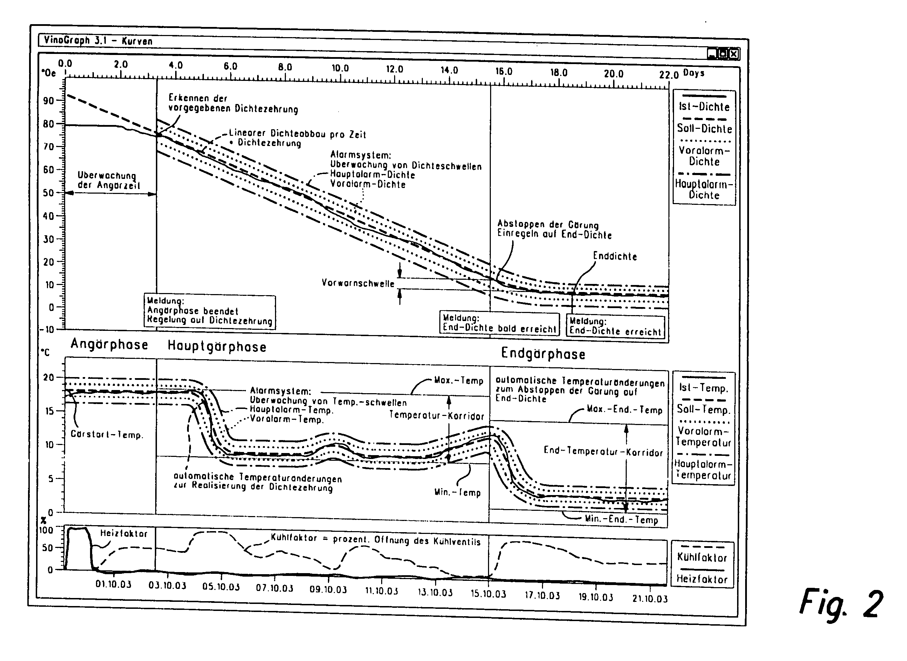

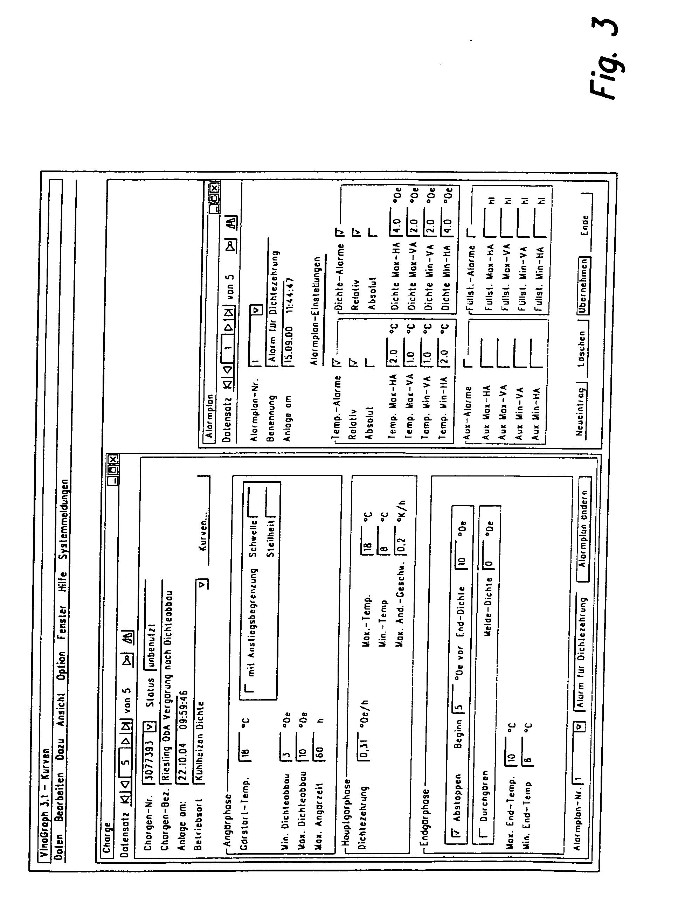

In Figur 2 sind die Verläufe von verschiedenen Signalen während der Ausführung des erfindungsgemäßen Verfahrens aufgetragen. Ein wesentlicher Aspekt der vorliegenden Erfindung ist darin zu sehen, dass die Temperatur der Flüssigkeit 3 zumindest während eines Teils des Gärprozesses derart geregelt wird, dass ein vorgebbarer zeitlicher Dichteverlauf der Flüssigkeit 3 möglichst genau eingehalten wird. In Figur 2 sind die Signalverläufe über der Zeit, im vorliegenden Fall über die Tage, aufgetragen. Der vorgegebene Soll-Dichteverlauf ist im oberen Teil der Figur 2 mit einer gestrichelten Linie dargestellt. Der Soll-Dichteverlauf wird im Vorfeld des Gärprozesses mathematisch oder empirisch ermittelt. Durch eine Variation des Soll-Dichteverlaufs, der erfindungsgemäß während des Gärprozesses abgefahren wird, kann die Qualität (Zusammensetzung, Aussehen, Geschmack, etc.) der Flüssigkeit 3 beeinflusst werden. Der Soll-Dichteverlauf wird von einem Anwender über den PC 6 vorgegeben und über die Datenleitung 8 an die SPS 7 übertragen. Die Vorgabe des Soll-Dichteverlaufs wird anhand der Figur 3 näher erläutert. Die SPS 7 regelt die Temperatur der Flüssigkeit dann so, dass der vorgegebene Dichteverlauf abgefahren wird. Der von der Dichtemesssonde 5 gemessene Wert für die Ist-Dichte ist mit einer durchgezogenen Linie dargestellt. Es ist deutlich zu erkennen, dass der Ist-Dichteverlauf im wesentlichen dem Soll-Dichteverlauf entspricht.

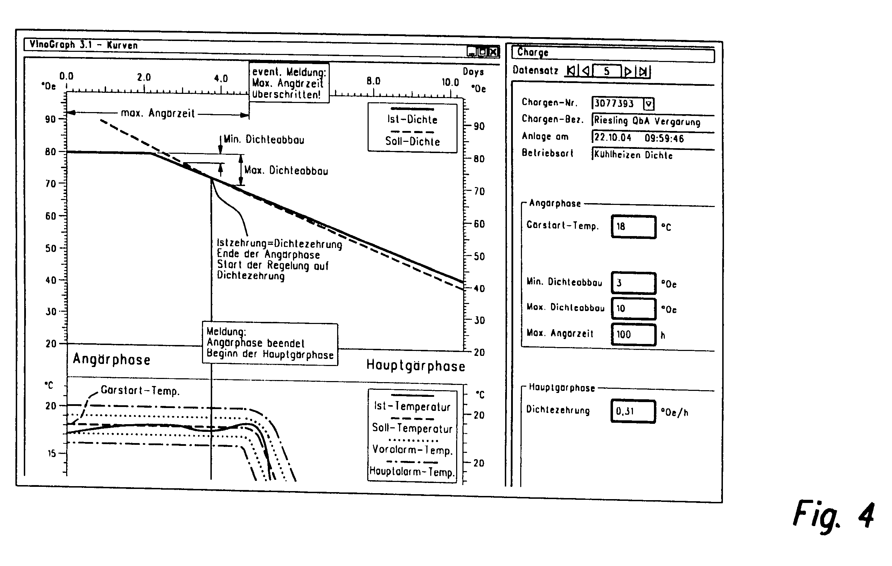

Außerdem kann Figur 2 entnommen werden, dass der Gärprozess in drei aufeinander folgende Abschnitte unterteilt ist, eine Angärphase, eine Hauptgärphase und eine Endgärphase. Selbstverständlich können während des Gärprozesses weitere Phasen durchlaufen werden. Die drei Phasen gemäß der beschriebenen Ausführungsform werden anhand der Figuren 4 bis 7 näher erläutert. In bestimmten Abständen zu dem Soll-Dichteverlauf oberhalb und unterhalb des Soll-Dichteverlaufs sind Dichteschwellen angegeben, deren Überschreiten (nach oben hin) bzw. Unterschreiten (nach unten hin) einen Alarm auslöst. Es sind dies zum einen eine näher an dem Soll-Dichteverlauf verlaufende Voralarm-Dichte (gepunktete Linie) und eine in einem größeren Abstand zu dem Soll-Dichteverlauf verlaufende Hauptalarm-Dichte (gestrichpunktete Linie).

Unterhalb der Dichteverläufe sind in Figur 2 die Temperaturverläufe der Flüssigkeit aufgetragen. Die Flüssigkeit 3 wird auf die dort dargestellten Werte geregelt, damit der in Figur 2 oben dargestellte Ist-Dichteverlauf der Flüssigkeit 3 erreicht wird. Der Soll-Temperaturverlauf ist mittels einer gestrichelten Linie dargestellt. Der Ist-Temperaturverlauf ist mit einer durchgezogenen Linie dargestellt. Ein Voralarm-Temperaturverlauf ist mit einer gepunkteten Linie dargestellt und ein Hauptalarm-Temperaturverlauf ist mit einer gestrichpunkteten Linie dargestellt. Außerdem ist bei den Temperaturverläufen die maximal und die minimal zulässige Temperatur der Flüssigkeit 3 eingezeichnet. Diese maximal und minimal zulässigen Temperaturen können für die verschiedenen Phasen des Gärprozesses unterschiedliche Werte annehmen.

Ganz unten ist in Figur 2 der Verlauf eines Heizfaktors (durchgezogene Linie) und eines Kühlfaktors (gestrichelte Linie) dargestellt. Diese Faktoren entsprechen im wesentlichen der Aktivierung der Ventile 9, 10 für die Heizmittel bzw. für die Kühlmittel während des Gärprozesses. Die nachfolgende qualitative Beschreibung der Kühl- und Heizfaktoren ist nicht allgemeingültig, da die Faktoren maßgeblich von den anlagenspezifischen Gegebenheiten abhängen und zum Teil stark von der nachfolgenden Beschreibung abweichen können. In dem dargestellten Ausführungsbeispiel ist deutlich zu erkennen, dass die Flüssigkeit 3 insbesondere zu Beginn der Angärphase (für etwa einen Tag) aufgeheizt wird (Heizfaktor nahe 100%), wohingegen während des übrigen Gärprozesses nur noch wenig geheizt wird (Heizfaktor von Zeit zu Zeit auf maximal 10%). Demgegenüber wird die Flüssigkeit 3 nach dem ersten Tag nahezu ständig gekühlt (Kühlfaktor zwischen 20% und 90%). Das hängt damit zusammen, dass die chemischen Reaktionen während des Gärprozesses exotherm sind, das heißt dass Wärme erzeugt wird.

In Figur 3 ist eine graphische Benutzerschnittstelle dargestellt, wie sie auf dem Bildschirm des PC 6 dargestellt wird. Über die Tastatur kann ein Anwender die gewünschten Werte für die angegebenen Parameter eingegeben. Über die eingegebenen Parameter ist der Soll-Dichteverlauf genau definiert. Die einzugegebenen Parameter umfassen zunächst allgemeine Angaben zu der gärenden Flüssigkeit, wie zum Beispiel Chargen-Nummer, Chargen-Bezeichnung, Datum des Gärprozesses. Für die Angärphase umfassen die Parameter die Gärstart-Temperatur (mit oder ohne Anstiegsbegrenzung), den minimalen und maximalen Dichteabbau und die maximale Angärzeit. Für die Hauptgärphase umfassen die einzugebenden Parameter zum Beispiel die gewünschte Dichtezehrung, die maximale und die minimale Temperatur der Flüssigkeit 3 und die maximale Temperatur-Änderungsgeschwindigkeit. Für die Endgärphase umfassen die Parameter die Auswahl einer von mehreren möglichen Arten der Endgärung (Abstoppen oder Durchgären), sowie die maximale und die minimale End-Temperatur. Außerdem können über die graphische Benutzerschnittstelle Parameter für einen sogenannten Alarmplan eingegeben werden, das heißt die Schwellen für den Voralarm und Hauptalarm können vorgegeben werden. Selbstverständlich ist es denkbar, dass der Anwender außer den genannten Parametern noch andere oder nur weniger Parameter eingeben kann. Schließlich ist es auch denkbar, eingegebene Parameter abzuspeichern, um dann zu einem späteren Zeitpunkt einfach einen bereits eingegebenen und abgespeicherten Soll-Dichteverlauf aufzurufen. Dadurch müssten nicht jedes Mal die Parameter für den gewünschten Gärprozess neu eingegeben werden.

In Figur 4 ist der Signalverlauf aus Figur 2 vergrößert im Ausschnitt für die Angärphase dargestellt. Während der Angärphase zu Beginn des Gärprozesses wird die Temperatur der Flüssigkeit auf einen vorgebbaren konstanten Wert (Gärstart-Temperatur) geregelt, bis sich in der Flüssigkeit 3 ein vorgebbarer Dichteabbau pro Zeiteinheit (zwischen minimalem Dichteabbau und maximalem Dichteabbau) einstellt oder bis eine vorgebbare Zeitdauer (maximale Angärzeit) überschritten ist. In der Angärphase greift das System, ausgehend von einer vorgebbaren, konstanten Gärstart-Temperatur zu einem bestimmten Zeitpunkt, bei dem eine optimale Dichtezehrung (Gärintensität) für die Einleitung der Hauptphase vorliegt, in den Gärprozess ein. Während der Angärphase wird der Dichtabbau überwacht und der optimale Zeitpunkt für die Einleitung der Hauptgärphase automatisch erkannt. Wird die optimale Dichtezehrung innerhalb einer vorgebbaren Zeitdauer nicht erreicht, wird nach Ablauf der Zeitdauer (maximale Angärzeit) trotzdem in die Hauptgärphase umgeschaltet.

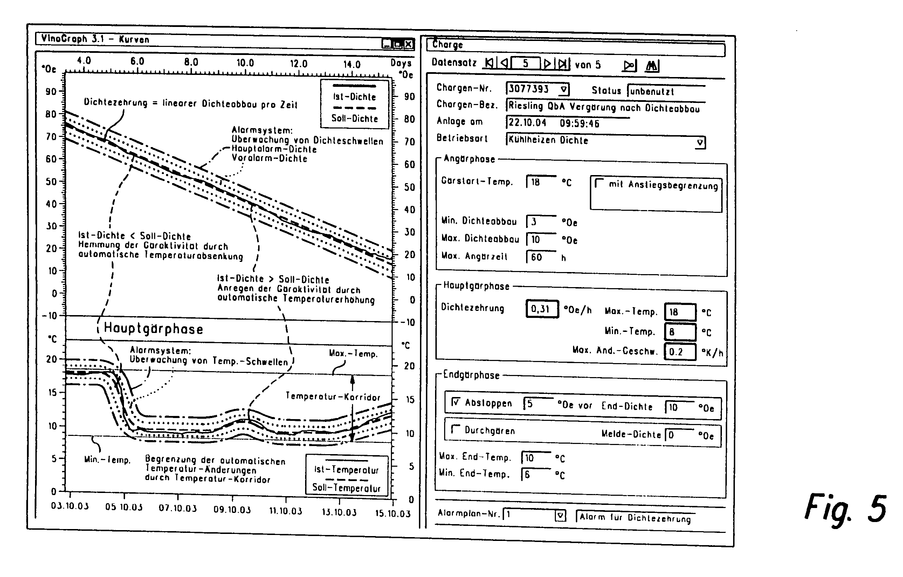

In Figur 5 ist der Signalverlauf aus Figur 2 vergrößert im Ausschnitt für die Hauptgärphase dargestellt. Während der Hauptgärphase wird die Temperatur der Flüssigkeit 3 derart geregelt, dass sich in der Flüssigkeit 3 ein vorgebbarer Dichteabbau pro Zeiteinheit (Dichtezehrung) einstellt. Während der Hauptgärphase ermöglichen automatische Temperaturänderungen der Flüssigkeit 3 eine schonende, vorgegebene Dichtezehrung. Neben der optimalen Streckung des Gärverlaufs zur Erzeugung bestimmter Weinstile ergibt sich dadurch eine planbare Gärdauer. Aus der Hauptgärphase wird automatisch in eine Endgärphase übergegangen, sobald ein vorgebbarer Schwellwert für die Dichte der Flüssigkeit 3 (End-Dichte plus Beginn vor End-Dichte) oder alternativ eine vorgebbare Zeitdauer überschritten ist.

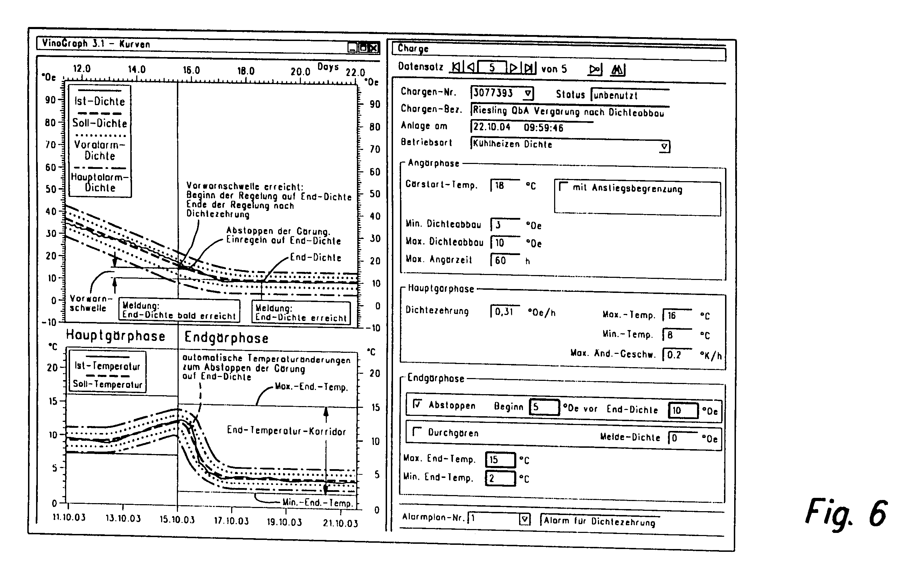

In Figur 6 ist der Signalverlauf aus Figur 2 vergrößert im Ausschnitt für eine erste Ausführungsform der Endgärphase dargestellt. Während der Endgärphase gegen Ende des Gärprozesses wird die Temperatur der Flüssigkeit 3 derart geregelt, dass sich in der Flüssigkeit 3 ein Dichteabbau pro Zeiteinheit (Dichtezehrung) von etwa Null einstellt und ein vorgebbarer Endwert (End-Dichte) für die Dichte der Flüssigkeit 3 erreicht wird. In der Endgärphase kann ein automatisches Abstoppen des Gärprozesses auf einen absoluten, vorgebbaren Dichte-Endwert (End-Dichte), beispielsweise zur Herstellung fructosebetonter Weine, durch geeignete Temperaturregelung erzielt werden. Die Temperaturregelung erfolgt dabei innerhalb eines Temperaturkorridors, der von dem zulässigen Temperaturbereich während der Angärphase und der Hauptgärphase abweichen kann. Dabei wird die Regelung nach Dichtezehrung beendet.

Die Temperatur der Flüssigkeit 3 wird während dieser Ausführungsform der Endgärphase innerhalb eines vorgebbaren End-Temperaturbereichs zwischen einer minimalen End-Temperatur und einer maximalen End-Temperatur geregelt. Vorzugsweise liegt dabei die minimale End-Temperatur niedriger als die minimale Temperatur während der Hauptgärphase und liegt die maximale End-Temperatur niedriger als die maximale Temperatur während der Hauptgärphase.

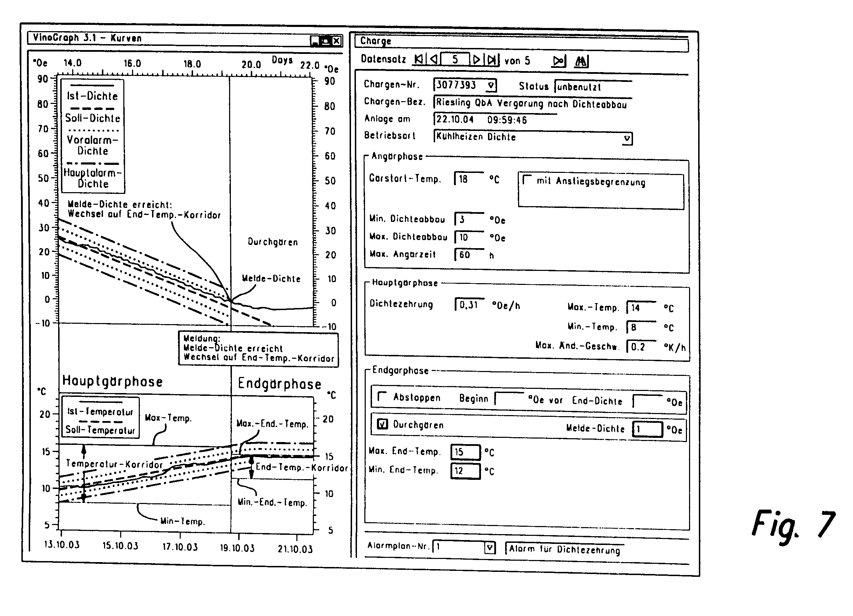

In Figur 7 ist der Signalverlauf aus Figur 2 vergrößert im Ausschnitt für eine alternative zweite Ausführungsform der Endgärphase dargestellt. Dabei wird die Temperatur der Flüssigkeit 3 derart geregelt, dass sich eine vollständige Durchgärung der Flüssigkeit 3 einstellt. Dabei wird mit der Dichtezehrung aus der Hauptgärphase mit denselben oder mit veränderten Parametern weitergearbeitet, das heißt die Regelung nach Dichtezehrung wird fortgesetzt.

Die Temperatur der Flüssigkeit 3 wird während der alternativen Ausführungsform der Endgärphase ebenfalls innerhalb des vorgebbaren End-Temperaturbereichs zwischen der minimalen End-Temperatur und der maximalen End-Temperatur geregelt. Vorzugsweise liegt dabei die minimale End-Temperatur höher als die minimale Temperatur während der Hauptgärphase, wohingegen die maximale End-Temperatur niedriger als die maximale Temperatur während der Hauptgärphase liegt.

Es versteht sich, dass der Soll-Dichteverlauf des erfindungsgemäßen Verfahrens von dem hier beispielhaft dargestellten Verlauf abweichen kann, beispielsweise um verschiedene Weinqualitäten zu erzielen. Außerdem ist der Einsatz des erfindungsgemäßen Verfahrens nicht auf die Herstellung von Wein oder Obstwein beschränkt, sondern kann für beliebige Gärprozesse, beispielsweise aus dem Bereich der Lebensmittel- oder Arzneimittelherstellung eingesetzt werden.

**Patentansprüche**

1. Verfahren zum kontrollierten Vergären einer Flüssigkeit (3), **dadurch gekennzeichnet, dass** die Temperatur der Flüssigkeit (3) zumindest während eines Teils des Gärprozesses derart geregelt wird, dass ein vorgebbarer zeitlicher Verlauf mindestens einer den Gärzustand der Flüssigkeit (3) charakterisierenden Größe zumindest annähernd eingehalten wird.

**2.** Verfahren nach Anspruch 1, **dadurch gekennzeichnet, dass** als eine den Gärzustand der Flüssigkeit (3) charakterisierende Größe die Dichte der Flüssigkeit (3) herangezogen wird.

**3.** Verfahren nach Anspruch 2, **dadurch gekennzeichnet, dass** die Dichte der Flüssigkeit (3) mittels einer Dichtemesssonde (5) gemessen wird.

**4.** Verfahren nach Anspruch 2, **dadurch gekennzeichnet, dass** die Dichte der Flüssigkeit (3) anhand anderer Größen ermittelt oder modelliert wird.

**5.** Verfahren nach Anspruch 1, **dadurch gekennzeichnet, dass** als eine den Gärzustand der Flüssigkeit (3) charakterisierende Größe der Kohlendioxid-Ausstoß der Flüssigkeit (3) herangezogen wird.

**6.** Verfahren nach einem der Ansprüche 1 bis 5, **dadurch gekennzeichnet, dass** die Temperatur der Flüssigkeit (3) innerhalb eines vorgebbaren Temperaturbereichs zwischen einer minimalen Temperatur und einer maximalen Temperatur geregelt wird.

**7.** Verfahren nach einem der Ansprüche 1 bis 6, **dadurch gekennzeichnet, dass** während einer Angärphase zu Beginn des Gärprozesses die Temperatur der Flüssigkeit (3) auf einen vorgebbaren konstanten Wert geregelt wird, bis sich in der Flüssigkeit (3) ein vorgebbarer Dichteabbau beziehungsweise Kohlendioxid-Strom pro Zeiteinheit einstellt, bis ein vorgebbarer absoluter Dichteabbau beziehungsweise Kohlendioxid-Strom stattgefunden hat.

**8.** Verfahren nach Anspruch 7, **dadurch gekennzeichnet, dass** nach der Angärphase automatisch in eine Hauptgärphase übergegangen wird, wobei die Temperatur der Flüssigkeit (3) während der Hauptgärphase derart geregelt wird, dass sich in der Flüssigkeit (3) ein vorgebbarer Dichteabbau beziehungsweise Kohlendioxid-Strom pro Zeiteinheit einstellt.

**9.** Verfahren nach Anspruch 8, **dadurch gekennzeichnet, dass** aus der Hauptgärphase automatisch in eine Endgärphase übergegangen wird, sobald ein vorgebbarer Schwellwert für die Dichte der Flüssigkeit (3) beziehungsweise für die aus der Flüssigkeit (3) abgegebene Kohlendioxid-Menge oder eine vorgebbare Zeitdauer überschritten ist.

**10.** Verfahren nach einem der Ansprüche 1 - 9, **dadurch gekennzeichnet, dass** während einer Endgärphase gegen Ende des Gärprozesses die Temperatur der Flüssigkeit (3) derart geregelt wird, dass sich in der Flüssigkeit (3) ein Dichteabbau beziehungsweise Kohlendioxid-Strom pro Zeiteinheit von etwa Null einstellt und ein vorgebbarer Endwert für die Dichte der Flüssigkeit (3) erreicht wird.

**11.** Verfahren nach Anspruch 10, **dadurch gekennzeichnet, dass** die Temperatur der Flüssigkeit (3) innerhalb eines vorgebbaren End-Temperaturbereichs zwischen einer minimalen End-Temperatur und einer maximalen End-Temperatur geregelt wird.

**12.** Verfahren nach Anspruch 11, **dadurch gekennzeichnet, dass** die minimale End-Temperatur niedriger ist als die minimale Temperatur während der Hauptgärphase und die maximale End-Temperatur niedriger ist als die maximale Temperatur während der Hauptgärphase.

**13.** Verfahren nach einem der Ansprüche 1 bis 12, **dadurch gekennzeichnet, dass** während einer Endgärphase gegen Ende des Gärprozesses die Temperatur der Flüssigkeit (3) derart geregelt wird, dass sich eine vollständige Durchgärung der Flüssigkeit (3) einstellt.

**14.** Verfahren nach Anspruch 13, **dadurch gekennzeichnet, dass** die Temperatur der Flüssigkeit (3) innerhalb eines vorgebbaren End-Temperaturbereichs zwischen einer minimalen End-Temperatur und einer maximalen End-Temperatur geregelt wird.

**15.** Verfahren nach Anspruch 14, **dadurch gekennzeichnet, dass** die minimale End-Temperatur gleich oder höher ist als die minimale Temperatur während der Hauptgärphase und die maximale End-Temperatur gleich oder niedriger ist als die maximale Temperatur während der Hauptgärphase.

**16.** Computerprogramm, das auf einem Rechengerät, insbesondere auf einem Mikroprozessor ablauffähig ist, **da-**

**durch gekennzeichnet, dass** das Computerprogramm zur Ausführung eines Verfahrens nach einem der Ansprüche 1 bis 15 programmiert ist.

17. Speicherelement, insbesondere Read-Only-Speicher, Random-Access-Speicher, Flash-Speicher oder Datenträger, **dadurch gekennzeichnet, dass** auf dem Speicherelement ein Computerprogramm abgespeichert ist, das zur Ausführung eines Verfahrens nach einem der Ansprüche 1 bis 15 programmiert ist.

18. Steuergerät (6, 7) für eine Temperaturregelung einer Flüssigkeit (3), umfassend ein Rechengerät, insbesondere einen Mikroprozessor, und ein Speicherelement, **dadurch gekennzeichnet, dass** auf dem Speicherelement ein Computerprogramm abgespeichert ist, das zur Ausführung eines Verfahrens nach einem der Ansprüche 1 bis 15 programmiert ist und das zur Ausführung des Verfahrens auf dem Rechengerät abläuft.

*Fig. 1*

Fig. 2

EP 1 559 775 A2

VinoGraph 3.1 - Kurven

Daten   Bearbeiten   Dazu   Ansicht   Option   Fenster   Hilfe   Systemmeldungen

**Charge**   _ ◘ ×

Datensatz  |◁ ◁ [ 5 ] ▷ ▷|  von 5      ▷◁  ⋔

Chargen-Nr.     [3077393] ▽   Status [unbenutzt]
Chargen-Bez.  [Riesling QbA Vergärung nach Dichteabbau]
Anlage am:     [22.10.04  09:59:46]
Betriebsart    [Kühlheizen Dichte                    ▽]      Kurven...

┌ Angärphase ───────
Gärstart-Temp.   [18]  °C      ☐ mit Anstiegsbegrenzung   Schwelle [ ]
                                                          Steilheit [ ]

Min. Dichteabbau   [3]   °Oe
Max. Dichteabbau   [10]  °Oe
Max. Angärzeit     [60]  h

┌ Hauptgärphase ───────
Dichtezehrung   [0,31] °Oe/h      Max.-Temp.        [18]  °C
                                  Min.-Temp         [8]   °C
                                  Max. And.-Geschw. [0,2] °K/h

┌ Endgärphase ───────
   ☑ Abstoppen    Beginn [5]  °Oe vor End-Dichte [10] °Oe
   ☐ Durchgären              Melde-Dichte [0] °Oe

Max. End-Temp.  [10]  °C
Min. End-Temp   [6]   °C

Alarmplan-Nr. [1]  ▽  [Alarm für Dichtezehrung]   Alarmplan ändern

**Alarmplan**   _ ◘ ×

Datensatz  |◁ ◁ [ 1 ] ▷ ▷|  von 5    ▷◁  ⋔

Alarmplan-Nr.   [1]        ▽
Benennung       [Alarm für Dichtezehrung]
Anlage am       [15.09.00  11:44:47]

Alarmplan-Einstellungen

┌Temp.-Alarme ☑ ───     ┌Dichte-Alarme ☑ ───
Relativ      ☑            Relativ      ☑
Absolut      ☐            Absolut      ☐

Temp. Max-HA [2.0] °C     Dichte Max-HA [4.0] °Oe
Temp. Max-VA [1.0] °C     Dichte Max-VA [2.0] °Oe
Temp. Min-VA [1.0] °C     Dichte Min-VA [2.0] °Oe
Temp. Min-HA [2.0] °C     Dichte Min-HA [4.0] °Oe

┌Aux-Alarme ☐ ───       ┌Füllst.-Alarme ☐ ───
Aux Max-HA [ ]           Füllst. Max-HA [ ] hl
Aux Max-VA [ ]           Füllst. Max-VA [ ] hl
Aux Min-VA [ ]           Füllst. Min-VA [ ] hl
Aux Min-HA [ ]           Füllst. Min-HA [ ] hl

Neueintrag | Löschen | Übernehmen | Ende

## Fig. 3

Fig. 4

VinoGraph 3.1 - Kurven

Dichtezehrung = linearer Dichteabbau pro Zeit

Ist-Dichte
Soll-Dichte

Alarmsystem:
Überwachung von Dichteschwellen
Hauptalarm-Dichte
Voralarm-Dichte

Ist-Dichte < Soll-Dichte
Hemmung der Garaktivität durch
automatische Temperaturabsenkung

Ist-Dichte > Soll-Dichte
Anregen der Garaktivität durch
automatische Temperaturerhöhung

Hauptgärphase

Alarmsystem:
Überwachung von Temp.-Schwellen

Max.-Temp.

Temperatur-Korridor

Min.-Temp.

Begrenzung der automatischen
Temperatur-Änderungen
durch Temperatur-Korridor

Ist-Temperatur
Soll-Temperatur

03.10.03  05.10.03  07.10.03  09.10.03  11.10.03  13.10.03  15.10.03

Charge

Datensatz  |◁  ◁  5  ▷  ▷|  von 5

Chargen-Nr.  3077393 ▽   Status  unbenutzt
Chargen-Bez.  Riesling QbA Vergärung nach Dichteabbau
Anlage am  22.10.04  09:59:46
Betriebsart  Kühlheizen Dichte  ▽

**Angärphase**

Garstart-Temp.  18  °C      mit Anstiegsbegrenzung

Min. Dichteabbau  3  °Oe
Max. Dichteabbau  10  °Oe
Max. Angärzeit  60  h

**Hauptgärphase**

Dichtezehrung  0,31  °Oe/h   Max.-Temp.  18  °C
Min.-Temp.  8  °C
Max. And.-Geschw.  0.2  °K/h

**Endgärphase**

☑ Abstoppen  5  °Oe vor End-Dichte  10  °Oe

☐ Durchgären      Melde-Dichte  0  °Oe

Max. End-Temp.  10  °C
Min. End-Temp.  6  °C

Alarmplan-Nr. 1  ▽  Alarm für Dichtezehrung

Fig. 5

EP 1 559 775 A2

VinoGraph 3.1 – Kurven

**Days**

Ist-Dichte
Soll-Dichte
Voralarm-Dichte
Hauptalarm-Dichte

Vorwarnschwelle erreicht:
Beginn der Regelung auf End-Dichte
Ende der Regelung nach
Dichtezehrung

Abstoppen der Gärung.
Einregeln auf End-Dichte

End-Dichte

Vorwarn-schwelle

Meldung:
End-Dichte bald erreicht

Meldung:
End-Dichte erreicht

Hauptgärphase    Endgärphase

Ist-Temperatur
Soll-Temperatur

automatische Temperaturänderungen
zum Abstoppen der Gärung
auf End-Dichte

Max.-End.-Temp.

End-Temperatur-Korridor

Min.-End.-Temp.

11.10.03    13.10.03    15.10.03    17.10.03    19.10.03    21.10.03

**Charge**

Datensatz  |◁ ◁ [ 5 ] ▷ ▷|  von 5

Chargen-Nr.  [3077393 ▽]    Status [unbenutzt]
Chargen-Bez.  [Riesling QbA Vergärung nach Dichteabbau]
Anlage am  [22.10.04  09:59:46]
Betriebsart  [Kühlheizen Dichte  ▽]

**Angärphase**

Gärstart-Temp.  [18] °C    ☐ mit Anstiegsbegrenzung

Min. Dichteabbau  [3] °Oe
Max. Dichteabbau  [10] °Oe
Max. Angärzeit  [60] h

**Hauptgärphase**

Dichtezehrung  [0,31] °Oe/h    Max.-Temp.  [16] °C
                                Min.-Temp.  [8] °C
                                Max. Änd.-Geschw.  [0.2] °K/h

**Endgärphase**

☑ Abstoppen    Beginn [5] °Oe vor End-Dichte [10] °Oe

☐ Durchgären    Melde-Dichte [0] °Oe

Max. End-Temp.  [15] °C
Min. End-Temp.  [2] °C

Alarmplan-Nr. [1]  [▽] [Alarm für Dichtezehrung]

*Fig. 6*

EP 1 559 775 A2

VinoGraph 3.1 - Kurven

°Oe  14.0    16.0    18.0    20.0  Days  22.0  °Oe

Ist-Dichte
Soll-Dichte
Voralarm-Dichte
Hauptalarm-Dichte

Melde-Dichte erreicht:
Wechsel auf End-Temp.-Korridor

Durchgären

Melde-Dichte

Meldung:
Melde-Dichte erreicht
Wechsel auf End-Temp.-Korridor

Hauptgärphase          Endgärphase

°C                          °C

Ist-Temperatur
Soll-Temperatur
Max-Temp.          Max.-End.-Temp.

Temperatur-Korridor    End-Temp.-Korridor

Min.-End.-Temp.

Min-Temp.

13.10.03  15.10.03  17.10.03  19.10.03  21.10.03

Charge

Datensatz  |◁ |◁  5  ▷| ▷|  von 5  ▷◁  ⋀

Chargen-Nr.    3077393  ▽    Status  unbenutzt
Chargen-Bez.   Riesling QbA Vergärung nach Dichteabbau
Anlage am      22.10.04   09:59:46
Betriebsart    Kühlheizen Dichte                ▽

Angärphase

Garstart-Temp.    18   °C        □ mit Anstiegsbegrenzung

Min. Dichteabbau   3   °Oe
Max. Dichteabbau  10   °Oe
Max. Angärzeit    60   h

Hauptgärphase

Dichtezehrung   0,31  °Oe/h     Max.-Temp.  14   °C
                                Min.-Temp.   8   °C
                           Max. Änd.-Geschw. 0.2  °K/h

Endgärphase

□ Abstoppen   Beginn       °Oe vor End-Dichte       °Oe

☑ Durchgären                    Melde-Dichte  1   °Oe

Max. End-Temp.   15   °C
Min. End-Temp.   12   °C

Alarmplan-Nr. 1       ▽   Alarm für Dichtezehrung

*Fig. 7*